Europäisches Patentamt

European Patent Office

Office européen des brevets

(11) Numéro de publication : **0 351 302 B1**

(12) **FASCICULE DE BREVET EUROPEEN**

(45) Date de publication du fascicule du brevet :
**10.03.93 Bulletin 93/10**

(51) Int. Cl.⁵ : **G01N 33/38, C04B 28/02**

(21) Numéro de dépôt : **89401980.1**

(22) Date de dépôt : **11.07.89**

(54) **Méthode de sélection d'une pouzzolane destinée à être incorporée dans un matériau composite comprenant du ciment et du verre.**

(30) Priorité : **13.07.88 FR 8809567**

(43) Date de publication de la demande :
**17.01.90 Bulletin 90/03**

(45) Mention de la délivrance du brevet :
**10.03.93 Bulletin 93/10**

(84) Etats contractants désignés :
**AT BE CH DE ES FR GB GR IT LI LU NL SE**

(56) Documents cités :
**EP-A- 0 252 848**
**US-A- 4 640 715**
**US-A- 4 642 137**
**CHEMICAL ABSTRACTS, vol. 102, no. 20, Columbus, OH (US); J.AMBROISE et al., p. 310, no. 171953z***
**CHEMICAL ABSTRACTS, vol. 98, no. 18, 1981, Columbus, OH (US); no. 148630z***

(73) Titulaire : **VETROTEX SAINT-GOBAIN**
**10, place Pierre de Coubertin**
**F-73000 Chambéry (FR)**

(72) Inventeur : **Soukatchoff, Pascal**
**4, Rue Louis Guingot**
**F-54690 Lay Saint-Christophe (FR)**
Inventeur : **Thiery, Jacques**
**6a, Rue Auguste Bichaton**
**F-54600 Villers Les Nancy (FR)**

(74) Mandataire : **Breton, Jean-Claude et al**
**SAINT-GOBAIN RECHERCHE 39, quai Lucien Lefranc**
**F-93300 Aubervilliers Cedex (FR)**

## Description

La présente invention concerne une méthode de sélection d'une pouzzolane destinée à être associée à du ciment, de la fibre de verre et, éventuellement d'autres constituants pour former un matériau composite.

L'invention est fondée sur la détermination de plusieurs caractéristiques physico-chimiques de la pouzzolane.

La présente invention trouve une application particulièrement importante, bien que non exclusive, dans le domaine des mortiers et bétons qui sont utilisés dans le bâtiment pour la fabrication de plaques ou panneaux devant bien résister au vieillissement (revêtement de façade, mobilier urbain, par exemple).

On connaît déjà le renforcement des liants minéraux du type ciment ou béton par des fibres de verre résistant aux alcalins. Lors de l'utilisation de telles fibres de verre, il se pose un problème de résistance et de durabilité du composant liant-fibres. Pour remédier à ce problème, une des solutions envisagées est d'ajouter des pouzzolanes à la matrice cimentaire.

Le terme de pouzzolane est utilisé ici dans le sens retenu par le brevet FR-A-2 149 998 : il s'agit de silicates, d'origine naturelle ou synthétique, susceptibles de réagir avec la chaux en se transformant en un matériau dur et résistant. Il peut s'agir de matières telles que les zéolites, les illites ou les métakaolins par exemple.

Si l'addition de pouzzolane au ciment renforcé de fibres de verre est bien connue, toutes les pouzzolanes ne confèrent pas au matériau composite final les mêmes propriétés mécaniques.

Afin de sélectionner les pouzzolanes susceptibles de conférer les meilleures propriétés mécaniques au matériau composite, il est déjà connu de mesurer leur surface spécifique et leur aptitude à fixer la chaux ; c'est le cas, notamment, des métakaolins.

Par métakaolin ou métakaolinite, il faut entendre le produit activé thermiquement de la kaolinite. La formule abrégée du métakaolin peut s'écrire, en utilisant les symboles classiques employés par les cimentiers : $AS_2$ ($A = Al_2O_3$ et $S = SiO_2$). Il s'obtient par traitement thermique de la kaolinite à des températures variant entre 700 et 900° C pendant des durées de quelques heures.

C'est ainsi que le brevet français FR-A-2 601 356 décrit des produits à base de ciment contenant des fibres de verre résistant aux alcalins et pour 100 parties en poids de ciment, d'environ 10 parties à environ 40 parties en poids de métakaolin. Le métakaolin entrant dans la composition de ce produit doit présenter une réactivité minimum mesurée selon l'essai Chapelle à 90° C, tel que défini dans l'article de BENOIT "Détermination de l'activité pouzzolanique d'une pouzzolane par voie chimique" - Bulletin de liaison des Laboratoires Routiers des Ponts et Chaussées n° 26 - 1967 pages D1 à D5, complété par l'article de R. LARGENT "Estimation de l'activité pouzzolanique - recherche d'un essai" - Bulletin de liaison des Laboratoires des Ponts et Chaussées n° 93 de janvier/février 1978, ref. 2143. Les métakaolins ainsi sélectionnés permettent de réaliser des produits qui présentent, en moyenne, de bonnes propriétés mécaniques. Toutefois, les mesures effectuées montrent que tous ces métakaolins ne présentent pas les mêmes avantages à long terme.

A la suite de nombreux tests effectués en laboratoire par les inventeurs, il est apparu que les caractéristiques physico-chimiques mesurées à ce jour ne permettent pas à coup sûr d'optimiser le choix du meilleur métakaolin ; il est donc apparu que la connaissance de ces caractéristiques (essai Chapelle à 90° C, surface spécifique par exemple) ne dispensait pas de mesurer les propriétés mécaniques du composite, de manière à affiner la sélection.

C'est pourquoi, les inventeurs ont recherché une méthode de sélection, fondée sur la mesure de caractéristiques physico-chimiques, qui réponde mieux que celles antérieurement connues aux exigences de la pratique, notamment en ce qu'elle permet de sélectionner de façon répétitive, immédiate et sûre, une pouzzolane (par exemple un métakaolin) permettant d'obtenir des produits présentant d'excellents résultats de résistance à la rupture immédiate et à terme, tout en garantissant une résistance, un maintien de la ductilité et de façon générale de bonnes propriétés mécaniques du matériau composite dans lequel la pouzzolane a été incorporée. L'invention vise notamment une méthode de sélection qui soit suffisamment sûre pour éviter les mesures sur composite, qui sont toujours longues et coûteuses, à long terme.

Les essais qui ont été réalisés et qui ont permis de mettre au point par étapes successives les mesures à effectuer pour obtenir des résultats optimaux ont été réalisés sur des composites à base de ciment renforcés par des fibres de verre alcali-résistant comprenant entre 20 et 30 % de métakaolin.

Dans ce but, l'invention propose une méthode de sélection d'une pouzzolane, telle qu'un métakaolin, destinée à être incorporée dans un matériau composite comprenant du ciment et des fibres de verre, qui consiste à mesurer, outre la surface spécifique de ladite pouzzolane et sa capacité à fixer la chaux selon l'essai Chapelle à 90° C, à mesurer sa capacité à fixer la chaux par un essai Chapelle réalisé à 50° C, et à calculer le rapport entre la quantité de chaux absorbée par gramme de pouzzolane avec l'essai Chapelle à 50° C et la surface spécifique de ladite pouzzolane, en vue de déduire, des différentes mesures et du rapport obtenus, la capacité de la pouzzolane à maintenir les propriétés mécaniques du matériau composite dans lequel elle est incorporée, après vieillissement.

Par essai Chapelle à 50° C, il faut entendre un essai Chapelle pratiqué à une température de l'ordre de 50° C pendant 16 heures.

Selon l'invention, on sélectionne la pouzzolane, telle qu'un métakaolin, qui présente une capacité à fixer la chaux à l'essai Chapelle à 90° C supérieure à 700 mg de CaO par gramme de pouzzolane ; une capacité à fixer la chaux à l'essai Chapelle à 50° C supérieure à 200 mg de CaO par gramme de pouzzolane ; et un rapport entre la quantité de chaux absorbée par gramme de pouzzolane avec l'essai Chapelle à 50° C et la surface spécifique du pouzzolane, supérieur à de l'ordre de 10 mg de CaO par mètre carré de pouzzolane et avantageusement supérieur à 12 mg de CaO par mètre carré de pouzzolane.

L'invention sera mieux comprise après l'exposé des résultats expérimentaux donnés et commentés ci-après obtenus à partir de différents métakaolins choisis à titre d'exemples. Afin d'établir les nouveaux éléments d'une méthode de sélection permettant de mieux caractériser les métakaolins efficaces pour les applications composites, il a été procédé à la formation de tels composites de la façon suivante :

- Le métakaolin est incorporé dans l'eau de gâchage en même temps que le ciment et le sable afin de former un mélange correspondant aux proportions suivantes :

```
- ciment           =           100 parties en poids,
- sable siliceux   =            50 parties en poids,
- métakaolin       entre 10 et 40 parties en poids,
- eau              entre 35 et 50 parties en poids,
- fluidifiant      =      de 1 à 3 parties en poids,
```

- le fluidifiant est choisi parmi les adjuvants couramment utilisés par l'homme du métier, tels que les sulfonates et les naphtalènes sulfonates.

- le composite est ensuite fabriqué à partir du mortier mouillé dont la composition est définie ci-dessus, avec un pourcentage de fibres de verre alcali-résistant de 3 à 6 % en poids.

De façon surprenante, en mesurant la réactivité du métakaolin à l'essai Chapelle à 50° C et en reliant cette réactivité pouzzolanique à la surface spécifique BET des grains de métakaolin, on s'aperçoit que plus le rapport obtenu est élevé, plus le vieillissement du matériau composite comprenant du verre et du métakaolin, tel que sélectionné, est limité, donc plus la ductilité est maintenue. L'essai Chapelle à 50° C et son rapport à la surface spécifique sont donc apparus aux inventeurs comme des mesures et calculs complémentaires indispensables et importants à prévoir dans le cas de la mesure des caractéristiques physico-chimiques d'un métakaolin.

Par ailleurs, à la suite des nombreux tests effectués sur différentes éprouvettes d'essais comprenant différents métakaolins et dont on a mesuré les caractéristiques physiques après vieillissement, il est apparu qu'un métakaolin présentant :

- une réactivité $Ic_{90}$ à l'essai Chapelle à 90° C supérieure à 700 mg de CaO par gramme de métakaolin,
- une réactivité $Ic_{50}$ à l'essai Chapelle à 50° C supérieure à 200 mg de CaO par gramme de métakaolin,
- un rapport $Ic_{50}$ sur la surface spécifique BET du métakaolin supérieur à 10 et de préférence 12 mg de CaO par mètre carré de métakaolin,

permettait d'obtenir des produits présentant les meilleurs résultats.

Enfin, les inventeurs ont pu observer que, pour une surface spécifique BET comprise entre 10 et 20 m² par gramme de métakaolin, de meilleurs résultats étaient également obtenus.

Plusieurs essais ont été réalisés, notamment à partir des métakaolins dont les spécifications sont données dans les tableaux I et II placés en annexe. (métakaolin symbolisé par MK).

Les composites ciment + métakaolin + verre alcali-résistant utilisés pour les essais et dont les résultats figurent au tableau III en annexe, ont été réalisés selon la procédure suivante :

- réalisation d'un mortier mouillé dont la composition est la suivante :

```
- CPA 55                    = 100      parties en poids
- sable siliceux (0-0,6 mm
  de granulométrie)         = 50       parties en poids
- métakaolin                = 22,5     parties en poids
```

EP 0 351 302 B1

```
- eau              =  40   à 46 parties en poids
- fluidifiant      =   1,5 à  3 parties en poids
```

- fabrication par projection de composites à partir de ce mortier avec un taux pondéral de l'ordre de 6 % en poids de fibre de verre alcali-résistant,
- mûrissement de 24 h sous bâche polyéthylène puis démoulage et mûrissement 27 jours à 20° C sous 98 % d'humidité relative, puis rectification et découpe des éprouvettes,
- vieillissement en eau chaude à 50° C jusqu'à 84 jours.

Ensuite, les échantillons sont cassés à l'état saturé (24 heures sous eau à 20° C) en flexion 3 points (élancement de 150 à 1 mm/mn) sur une machine universelle, du type connu sous la dénomination INSTRON.

Les métakaolins du tableau III sont ceux définis dans les tableaux I et II. Les paramètres LOP (Limit of Proportionality) ; MOR (Modulus of Rupture) et ε (allongement à la rupture) sont définis dans la norme anglaise BS 64-32 de 1984.

Les ciments utilisables avec l'invention sont notamment du type ciment Portland classique CPA, mais tous types de ciments utilisés pour les ciments renforcés à la fibre de verre (GRC) sont possibles.

Les compositions des verres alcali-résistants utilisés sont notamment des compositions présentant plus de 12 % (en poids) de $ZrO_2$, et même avantageusement plus de 15 %.

Les métakaolins n° 1 et 2 relèvent de la présente invention, à savoir qu'ils présentent des caractéristiques répondant aux spécifications précédemment énoncées, c'est-à-dire :
- une surface spécifique BET comprise entre 10 et 20 m² par gramme,
- des indices Chapelle à 50° C et 90° C respectivement supérieurs à 200 mg et 700 mg de CaO par gramme de métakaolin,
- un rapport $Ic_{50}$ sur BET supérieur à 12 mg de CaO par mètre carré de métakaolin.

Au contraire, les métakaolins 3, 4 et 5 ne répondent pas dans leur intégralité aux quatre critères de sélection:
- le métakaolin n° 3 ne répond à aucun des critères,
- le métakaolin n° 4 ne satisfait pas aux critères sur la surface spécifique et au critère sur le rapport de réactivité à l'essai Chapelle $Ic_{50}$ sur la surface spécifique du métakaolin BET,
- le métakaolin n° 5 ne satisfait pas le critère relatif à l'essai Chapelle à 90° C.

On peut constater que, après 28 jours de mûrissement à 20° C et 98 % d'humidité relative, les valeurs des LOP, MOR et EPS (ε) sont comparables pour les métakaolins n° 1, 2, 3 et 5. Par contre, le métakaolin n° 4 peut conduire à des résistances mécaniques plus faibles compte tenu de sa surface BET trop élevée.

Après 84 jours de vieillissement sous eau à 50° C, on constate par contre, une nette supériorité des composites fabriqués avec les métakaolins n° 1 et 2, surtout eu égard aux paramètres EPS (ε) et MOR. Pour ces deux métakaolins, la valeur à terme de EPS (ε) est de 0,7 %, ce qui permet d'assurer au matériau composite comprenant du verre alcali-résistant un maintien de leur ductilité et de leurs énergies à la rupture.

En revanche, les composites fabriqués au moyen des métakaolins n° 3, 4 et 5 présentent des chutes de ductilité (ε) d'au moins 50 %. Ceci fait apparaître les avantages de la méthode de sélection de l'invention. En effet, sans les nouveaux critères de l'invention, les métakaolins n° 3, 4 et 5 auraient pu être sélectionnés, à tort. En particulier, le métakaolin n° 4 qui présente de bons résultats selon l'art antérieur est à éliminer du fait de son rapport $Ic_{50}$/BET trop faible, le métakaolin n° 3 de sa capacité insuffisante à fixer la chaux, aussi bien à 50° C qu'à 90° C et le métakaolin n° 5 d'une capacité à fixer la chaux également insuffisante à 90° C lié à une surface spécifique un peu trop élevée.

Grâce à la présente invention, il est donc possible de sélectionner une pouzzolane destinée à être incorporée dans un composite ciment-fibres de verre, de manière à lui conférer une ductilité suffisante à terme, supérieure à 0,5 %, ainsi que des résistances mécaniques optimales. Des composites à base de ciment classique du type CPA, comprenant de la fibre de verre et présentant une telle ductilité, ne sont pas connus de l'art antérieur. De tels résultats à l'allongement ne sont en effet obtenus, après 28 jours + 84 jours, qu'avec des ciments alumineux, présentant les inconvénients de subir un changement allotropique dans le temps et d'être obligatoirement teintés en gris, et avec des ciments sur-sulfatés $C_4A_3\overline{S}$ dits "Chichibu" très coûteux, présentant également un aspect obligatoirement teinté, c'est-à-dire non blanc. Ces ciments présentent des inconvénients, tels que des conditions sévères de cure (température et humidité), à la différence du produit suivant l'invention. Les composites réalisés à partir de pouzzolanes sélectionnées selon la présente invention n'évoluent donc quasiment pas dans le temps.

La sûreté de la méthode selon l'invention permet de retenir ou d'écarter une pouzzolane, sans qu'il soit nécessaire de procéder aux différentes mesures sur composite. Cette sûreté permet un gain de temps et une

4

économie des moyens mis en oeuvre tout à fait remarquables.

## T A B L E A U   I

| : | : $SiO_2$ % | $Al_2O_3$ % | $Fe_2O_3$ % | $TiO_2$ % | CaO % | MgO % | $K_2O$ % | $Na_2O$ % | Perte feu % |
|---|---|---|---|---|---|---|---|---|---|
| :MK n°1: | 59,65 | 36,08 | 1,93 | 0,25 | 0,29 | 0,15 | 0,41 | 0,02 | 1,37 |
| :MK n°2: | 55,47 | 39,65 | 0,62 | 0,02 | 0,07 | 0,23 | 2,87 | 0,07 | 0,88 |
| :MK n°3: | 54,72 | 42,18 | 0,57 | 0,01 | 0,05 | 0,20 | 1,46 | 0,05 | 0,41 |
| :MK n°4: | 51,23 | 40,27 | 2,29 | 2,38 | 0,73 | 0,16 | 0,03 | 0,03 | 2,25 |
| :MK n°5: | 51,48 | 43,26 | 1,17 | 1,37 | 0,32 | 0,05 | 0,5 | 0,04 | 1,39 |

## T A B L E A U   II

| : | surface BET $m^2/g$ | Ic essais Chapelle mg CaO/g Métakaolin | | Ic50/BET mg CaO par $m^2$ de métakaolin |
|---|---|---|---|---|
| : | : | $Ic_{50}$ | $Ic_{90}$ | : |
| :MK n°1: | 17 | 260 | 770 | 15,3 |
| :MK n°2: | 14,8 | 430 | 820 | 29 |
| :MK n°3: | 8,3 | 100 | 570 | 12 |
| :MK n°4: | 48,6 | 430 | 870 | 8,8 |
| :MK n°5: | 24 | 240 | 630 | 10 |

# T A B L E A U   III

| | M 28 | | | M 28 + 28 EC | | |
|---|---|---|---|---|---|---|
| | LOP | MOR | ε | LOP | MOR | ε |
| 22,5 % métakaolin n°1 | 12 | 28 | 0,94 | 13 | 29 | 0,80 |
| 22,5 % métakaolin n°2 | 12 | 28 | 0,80 | 14 | 31 | 0,80 |
| 22,5 % métakaolin n°3 | 14 | 27 | 0,9 | 15 | 31 | 0,6 |
| 22,5 % métakaolin n°4 | 9 | 20 | 0,8 | 13 | 25 | 0,7 |
| 22,5 % métakaolin n°5 | 11 | 29 | 0,97 | 13 | 21 | 0,4 |

## Suite du TABLEAU III

| | M 28 + 56 EC | | | M 28 + 84 EC | | |
|---|---|---|---|---|---|---|
| | LOP | MOR | ε | LOP | MOR | ε |
| 22,5 % métakaolin n°1 | 12 | 28 | 0,70 | 13 | 29 | 0,70 |
| 22,5 % métakaolin n°2 | 14 | 30 | 0,7 | 15 | 30 | 0,70 |
| 22,5 % métakaolin n°3 | 15 | 31 | 0,60 | 16 | 28 | 0,40 |
| 22,5 % métakaolin n°4 | 13 | 23 | 0,50 | 14 | 23 | 0,40 |
| 22,5 % métakaolin n°5 | 13 | 20 | 0,25 | 14 | 17 | 0,15 |

LOP et MOR en MPa

ε en %

**Revendications**

1. Méthode de sélection d'une pouzzolane, telle qu'un métakaolin, destinée à être incorporée dans un matériau composite comprenant du ciment et des fibres de verre, qui consiste à mesurer, outre la surface spécifique de ladite pouzzolane et sa capacité à fixer la chaux selon l'essai Chapelle à 90° C, sa capacité à fixer la chaux par un essai Chapelle à 50° C, puis à calculer le rapport entre la quantité de chaux absorbée par gramme de pouzzolane lors de l'essai Chapelle à 50° C et la surface spécifique de ladite pouzzolane, en vue de déduire, des différentes mesures et du rapport obtenus, la capacité de la pouzzolane à maintenir les propriétés mécaniques du matériau composite dans lequel elle est incorporée, après vieillissement.

2. Méthode de sélection selon la revendication 1, **caractérisée en ce qu'**on retient la pouzzolane qui présente une capacité à fixer la chaux à l'essai Chapelle à 90° C supérieure à 700 mg de CaO par gramme de pouzzolane, une capacité à fixer la chaux à l'essai Chapelle à 50° C supérieure à 200 mg de CaO par gramme de pouzzolane, un rapport, entre la quantité de chaux absorbée par gramme de pouzzolane avec l'essai Chapelle à 50° C et la surface spécifique de la pouzzolane, supérieur à de l'ordre de 10 mg de CaO par mètre carré de pouzzolane et avantageusement supérieur à 12 mg de CaO par mètre carré de pouzzolane.

3. Méthode de sélection selon la revendication 2, **caractérisée en ce qu'**on sélectionne la pouzzolane dont la surface spécifique est comprise entre 10 et 20 $m^2$ par gramme.

**Patentansprüche**

1. Verfahren zur Auswahl eines Puzzolans, etwa eines Metakaolins, das dazu bestimmt ist, in ein Zement und Glasfasern enthaltenes Verbundmaterial eingebracht zu werden, das darin besteht, neben der spezifischen Oberfläche des Puzzolans und seinem Kalkbindevermögen nach Chapelle bei 90° C, sein Kalkbindevermögen nach Chapelle bei 50° C zu messen, danach das Verhältnis zwischen der pro Gramm Puzzolan nach Chapelle bei 50° C absorbierten Kalkmenge und der spezifischen Oberfläche des Puzzolans zu berechnen, um aus den verschiedenen Messungen und dem erhaltenen Verhältnis die Fähigkeit des Puzzolans herzuleiten, die mechanischen Eigenschaften des Verbundmaterials, in das es eingebracht wird, nach der Alterung zu behalten.

2. Auswahlverfahren nach Anspruch 1, dadurch gekennzeichnet, daß man ein Puzzolan auswählt, das ein Kalkbindevermögen nach Chapelle bei 90° C von mehr als 700 mg CaO pro Gramm Puzzolan, ein Kalkbindevermögen nach Chapelle bei 50° C von mehr als 200 mg CaO pro Gramm Puzzolan, ein Verhältnis zwischen der Menge an adsorbiertem Kalk pro Gramm Puzzolan nach Chapelle bei 50° C und der spezifischen Oberfläche des Puzzolans von mehr als, in der Größenordnung, 10 mg CaO pro $m^2$ Puzzolan und vorzugsweise mehr als 12 mg CaO pro$^2$ Puzzolan aufweist.

3. Auswahlverfahren nach Anspruch 2, dadurch gekennzeichnet, daß man ein Puzzolan auswählt, dessen spezifische Oberfläche zwischen 10 und 20 $m^2$ pro Gramm liegt.

**Claims**

1. Method of selecting a pozzuolana such as metakaolin which is intended to be incorporated into a composite material comprising cement and glass fibres, which consists in measuring in addition to the specific surface area of the said pozzuolana and its capacity to fix lime according to the Chapelle test at 90°C, its capacity to fix lime by a Chapelle test at 50°C, then calculating the ratio between the quantity of lime absorbed per gram of pozzuolana during the Chapelle test at 50°C and the specific surface area of the said pozzuolana, with a view to deducing from the various measurements and from the ratio obtained, the capacity of the pozzuolana to maintain the mechanical properties of the composite material in which it is incorporated, after ageing.

2. A method of selection according to Claim 1, characterised in that the pozzuolana is chosen which offers a capacity to fix lime in the Chapelle test at 90°C which is greater than 700 mg CaO per gram of pozzuolana, a capacity to fix lime in the Chapelle test at 50°C which is greater than 200 mg CaO per gram of pozzuolana, a ratio between the quantity of lime absorbed per gram of pozzuolana with the Chapelle test at 50°C and the specific surface area of the pozzuolana which is greater than approximately 10 mg CaO per $m^2$ of pozzuolana and advantageously greater than 12 mg CaO per $m^2$ of pozzuolana.

3. A method of selection according to Claim 2, characterised in that the specific surface area of the pozzuolana selected is between 10 and 20 $m^2/g$.